# EUROPÄISCHE PATENTANMELDUNG

(11) **EP 4 529 225 A1**
(43) Veröffentlichungstag der Anmeldung: **26.03.2025**
(21) Anmeldenummer: 24195494.0
(22) Anmeldetag: 20.08.2024
(51) Int. Cl.: H04R 5/04, H04R 1/10, H04R 25/00, A61M 21/02, A61B 5/38, A61B 5/00

(54) **HÖRSYSTEM SOWIE VERFAHREN ZUR ERZEUGUNG VON MONAURALEN ODER BINAURALEN BEATS**

(30) Priorität: 19.09.2023 DE 102023209095
(71) Anmelder: Sivantos Pte. Ltd., Singapore 539775 (SG)
(72) Erfinder: FRÖHLICH, Matthias, 91058 Erlangen (DE); HAIN, Jens, 91058 Erlangen (DE); HÖGL, Florian, 91058 Erlangen (DE); NAUMANN, Frank, 91058 Erlangen (DE)
(74) Vertreter: FDST Patentanwälte

(57) **Zusammenfassung**

Bei dem Hörsystem (2) handelt es sich insbesondere um ein binaurales Hörsystem (2), welches zur Erzeugung von monauralen oder binauralen Beats ausgebildet ist und hierzu zumindest ein Hörgerät (4A, 4B) mit einem Haupt-Signalpfad (8) aufweist. Das Hörgerät (4A, 4B) weist weiter einen Neben-Signalpfad (26) auf, mit einem Signalgenerator (28) zur Erzeugung eines ersten Beatsignals (B1), wobei das erste Beatsignal (B1) zu einem weiteren Signal (B2) um eine Beatfrequenz frequenzverschoben ist, derart, dass im Betrieb beim Nutzer der Effekt des monauralen oder binauralen Beats erzeugt wird. Dem Signalgenerator (28) ist eine Einstellvorrichtung (36) zugeordnet, die ein einstellbares Verstärkerelement (34) aufweist, wobei die Einstellvorrichtung (36) eine Analyseeinheit (32) zur Bestimmung eines momentanen Signalpegels (Lₘ) des Eingangssignals (E1, E2) aufweist, und wobei die jeweilige Einstellvorrichtung (36) dazu eingerichtet ist, einen Signalpegel (L_{B}) des ersten Beatsignals (B1) in Abhängigkeit des momentanen Signalpegels (Lₘ) einzustellen.

## Beschreibung

Die Erfindung betrifft ein insbesondere binaurales Hörsystem sowie ein Verfahren zur Erzeugung von monauralen oder binauralen Beats mithilfe eines derartigen Hörsystems.

Unter Hörsystem wird vorliegend ein System mit zumindest einem Hörgerät und bei einem binauralen Hörsystem ein System mit zwei Hörgeräten verstanden. Bei den Hörgeräten handelt es sich um elektronische Geräte, die ein Nutzer auf dem Ohr, hinter dem Ohr oder im Ohr trägt. Bei dem Hörsystem handelt es sich insbesondere um ein Hörhilfesystem, welches dazu eingerichtet ist, einen nutzerspezifischen Hörschaden einer hörgeschädigten Person zu kompensieren.

Ein derartiges Hörgerät weist jeweils üblicherweise einen Eingangswandler auf, welcher ein akustisches oder auch ein gestreamtes Eingangssignal zunächst in ein elektrisches Eingangssignal wandelt, welches nachfolgend mittels einer insbesondere digitalen Signalverarbeitungseinheit zu einem elektrischen Ausgangssignal aufbereitet wird, welches einem Ausgangswandler zugeführt wird, welcher das elektrische Ausgangssignal in ein akustisches Ausgangssignal wandelt, welches an das Ohr des Hörers abgegeben wird.

Unter monauralen Beats oder binauralen Beats werden bekannte Effekte einer Sinneswahrnehmung im menschlichen Gehirn verstanden, bei der der Nutzer einen pulsierenden Ton (Beat) wahrnimmt, wenn ihm zwei gleiche akustische Signale mit leicht voneinander abweichender Frequenz dargeboten werden. Bei binauralen Beats werden dem Nutzer auf zwei Kanälen, nämlich auf dem linken Ohr und auf dem rechten Ohr, die beiden zueinander frequenzverschobenen akustischen Signale dargeboten. Bei monauralen Beats werden die beiden frequenzverschobenen akustischen Signale lediglich auf einem Kanal dargeboten.

Mit derartigen monauralen oder binauralen Beats lassen sich bestimmte Trainingseffekte für das Gehirn erreichen.

Aus der EP 2 671 390 B1 ist ein binaurales Hörhilfesystemen zur Erzeugung von binauralen Beats zu entnehmen. Bei diesem weisen die beiden Hörgeräte miteinander synchronisierte Soundgeneratoren auf, welche jeweils ein Audiosignal erzeugen, wobei eines dieser Audiosignale in dem einen Hörgerät frequenzverschoben wird. Die Audiosignale werden jeweils über einen Summierer dem elektrischen Eingangssignal aufsummiert.

Ausgehend hiervon liegt der Erfindung die Aufgabe zugrunde, mit Hilfe eines Hörsystems akustische Ausgangssignal zu erzeugen, die geeignet sind, beim Benutzer eine angenehme Wahrnehmung von monauralen oder binauralen Beats zu erzeugen.

Die Aufgabe wird gemäß der Erfindung gelöst durch ein Hörsystem, insbesondere ein binaurales Hörsystem mit den Merkmalen des Anspruchs 1 sowie durch ein Verfahren zur Erzeugung von monauralen oder binauralen Beats mithilfe eines solchen Hörsystems. Im Hinblick auf das Hörsystem angeführte Vorteile und bevorzugten Ausgestaltungen sind sinngemäß auch auf das Verfahren und umgekehrt zu übertragen.

Bei dem Hörsystem handelt es sich insbesondere um ein Hörhilfesystem, welches nutzerspezifisch eingerichtet ist und zur Kompensation eines nutzerspezifischen Hörschadens ausgebildet ist.

Das Hörsystem weist zumindest ein Hörgerät und bevorzugt, speziell bei der Ausgestaltung als binaurales Hörsystems, zwei Hörgeräte auf, wobei
- das zumindest eine Hörgerät einen Haupt-Signalpfad aufweist, mit einem Eingangswandler zur Erzeugung eines elektrischen Eingangssignals aus einem beispielsweise akustischen oder gestreamten Eingangssignal, mit einer insbesondere digitalen Signalverarbeitungseinheit zur Verarbeitung des elektrischen Eingangssignals und zur Erzeugung eines elektrischen Ausgangssignals, sowie mit einem Ausgangswandler, welcher basierend auf dem elektrischen Ausgangssignal ein akustisches Ausgangssignal erzeugt,
- das zumindest eine Hörgerät weiterhin einen Neben-Signalpfad aufweist, mit jeweils einem Signalgenerator zur Erzeugung eines ersten Beatsignals, wobei das erste Beatsignal zu einem weiteren Signal um eine Beatfrequenz frequenzverschoben ist, derart, dass im Betrieb beim Nutzer der Effekt des monauralen oder binauralen Beats erzeugt wird,
- das zumindest eine Hörgerät einen Summierer aufweist, welcher zumindest das erste Beatsignal auf ein Signal des Haupt-Signalpfades aufsummiert, so dass das akustische Ausgangssignal gebildet ist durch einen normalen Audiosignalanteil und einen Beatsignalanteil,
- dem Signalgenerator eine Einstellvorrichtung zugeordnet ist, die ein einstellbares Verstärkerelement aufweist, wobei die Einstellvorrichtung eine Analyseeinheit zur Bestimmung eines momentanen Signalpegels des Eingangssignals aufweist, und dass die Einstellvorrichtung dazu eingerichtet ist, einen Signalpegel des Beatsignals in Abhängigkeit des momentanen Signalpegels einzustellen.

Im Falle eines binauralen Hörsystems weisen die beiden Hörgeräte jeweils eine Kommunikationseinheit auf, die eine Kommunikationsschnittstelle bildet, über die die beiden Hörgeräte in bekannter Weise wechselweise miteinander kommunizieren und Daten austauschen. Beispielsweise ist hierzu eine Bluetooth-Verbindung und insbesondere eine NFMI-Verbindung ausgebildet.

Bei einem Hörsystem mit zwei Hörgeräten, insbesondere bei einem binauralen Hörsystem, weist das zweite Hörgerät ebenfalls einen Haupt-Signalpfad mit den zuvor aufgeführten Komponenten auf.

In bevorzugter Ausgestaltung, jedoch nicht zwingend, weist ein derartiges zweites Hörgerät auch einen Neben-Signalpfad auf, in dem ebenfalls ein Signalgenerator zur Erzeugung eines zweiten Beatsignals angeordnet ist. In dieser Ausführungsvariante sind die beiden Beatsignale um die Beatfrequenz zueinander frequenzverschoben. Auch das zweite Hörgerät weist in diesem Fall einen Summierer auf, welcher das zweite Beatsignal auf ein Signal des ersten Signalpfades aufsummiert. Dadurch wird auch im zweiten Hörgerät ein akustisches Ausgangssignal erzeugt, welches gebildet ist durch einen normalen Audiosignalanteil und einen Beatsignalanteil auf Basis des zweiten Beatsignals.

Unter normalen Audiosignalanteil wird im akustischen Ausgangssignal derjenige Signalanteil verstanden, welcher sich ohne die Aufsummierung des Beatsignals ergeben würde.

Weiterhin ist bevorzugt auch dem Signalgenerator des zweiten Hörgeräts eine Einstellvorrichtung zugeordnet mit einem einstellbaren Verstärkerelement sowie mit einer Analyseeinheit zur Bestimmung des momentanen Signalpegels des Eingangssignals am zweiten Hörgerät. Der Signalpegel des zweiten Beatsignals wird in Abhängigkeit des momentanen Signalpegels des Eingangssignals des zweiten Hörgeräts eingestellt. Bei einer bevorzugten Variante wird daher in beiden Hörgeräten mit der Analyseeinheit jeweils ein momentaner Signalpegel des Eingangssignals des jeweiligen Eingangswandlers bestimmt.

Alternativ hierzu besteht auch die Möglichkeit, insbesondere bei einem binauralen Hörsystem, dass lediglich ein Hörgerät eine Analyseeinheit aufweist bzw. lediglich ein Hörgerät den momentanen Signalpegel bestimmt und diesen an das andere Hörgerät über die Kommunikationsschnittstelle übermittelt.

Durch die Signalpegel-abhängige Einstellung des Signalpegels des Beatsignals wird insgesamt die Amplitude und damit die Lautstärke des jeweiligen Beatsignals quasi dem momentanen Signalpegel des Eingangssignals nachgeführt. Der Signalpegel des Beatsignals wird daher insbesondere anhand der von dem zumindest einen Eingangswandler aufgenommenen Umgebungsgeräuschen eingestellt und nachgeführt. Hierdurch wird beim Nutzer eine angenehme Wahrnehmung der monauralen oder binauralen Beats erzeugt.

Die Ausgestaltung eines Neben-Signalpfades beim zweiten Hörgerät ist nicht zwingend erforderlich, auch nicht bei binauralen Hörgeräten und / oder bei der Erzeugung von binauralen Beats.

Im Fall der Erzeugung von monauralen Beats wird beispielsweise lediglich innerhalb des zumindest einen Hörgerätes ein normaler Signalanteil und ein frequenzverschobener Signalanteil bereitgestellt, die dann zur Wahrnehmung der monauralen Beats führen. Auch bei der Erzeugung von binauralen Beats ist es in einer Ausführungsvariante ausreichend, wenn beispielsweise im Signalgenerator des ersten Hörgeräts das Beatsignal auf Basis des Eingangssignals beispielsweise lediglich durch eine Frequenzverschiebung des Eingangssignals oder von Signalanteilen des Eingangssignals erzeugt wird. Der binaurale Effekt wird beispielsweise durch das Audiosignal (akustisches Ausgangssignal) am zweiten Hörgerät und dem durch den frequenzverschobenen Beatsignalanteil (auf Basis des ersten Beatsignals) im akustischen Ausgangssignal des ersten Hörgeräts erzeugt.

Insbesondere bei solchen Varianten wird im zweiten Hörgerät auf einen Neben-Signalpfad verzichtet und / oder es wird lediglich im ersten Hörgerät ein frequenzverschobenes Beatsignal erzeugt.

Bevorzugt ist jedoch die Variante, bei dem beide Hörgeräte einen Signal-Nebenpfad und jeweils einen Signalgenerator aufweisen.

Sofern vorliegend von einem Beatsignal gesprochen wird, so wird hierunter allgemein ein Signal verstanden, welches im akustischen Ausgangssignal, also dem ausgegebenen Audiosignal zu einem Signalanteil beiträgt, welcher zur Erzeugung des gewünschten Effekts des monauralen oder binauralen Beats beiträgt.

Bevorzugt wird im jeweiligen Signalgenerator zusätzlich zum Eingangssignal ein zusätzliches, weiteres Signal erzeugt, welches auch als Trägersignal bezeichnet wird und welches vorzugsweise unabhängig vom Eingangssignal ist. Hierbei handelt es sich beispielsweise um einen Ton (z.B. Sinussignal) oder auch um ein insbesondere schmalbandiges Rauschen.

Wird kein zusätzliches Signal erzeugt und die Beats beispielsweise lediglich durch das normale Audiosignal und einen hierzu frequenzverschobenen Audiosignalanteil erzeugt, so handelt es sich bei dem normalen Audiosignal und dem hierzu frequenzverschobenen Audiosignalanteil um die beiden Beatsignale, die zur Wahrnehmung der Beats beim Nutzer führen.

Bei dem weiteren Signal, zu dem das erste Beatsignal um eine Beatfrequenz verschoben ist, handelt es sich insbesondere um das vom zweiten Signalgenerator erzeugte zweite Beatsignal. Alternativ handelt es sich um das elektrische Eingangssignal oder einem hieraus abgeleiteten Signalanteil. Bei dieser alternativen Variante wird also das erfasste Umgebungsgeräusch für die Erzeugung der Beatsignale herangezogen.

Unter Beatfrequenz wird vorliegend die Frequenz verstanden, um die die beiden Beatsignale zueinander frequenzverschoben sind. Die Beatfrequenz liegt dabei typischerweise im Bereich von 0,5 Hz bis 60 Hz, vorzugsweise maximal bis 55 Hz und häufig im Bereich zwischen 3 Hz und 30 Hz.

Zur Erzeugung des frequenzverschobenen Beatsignals weist zumindest der eine Signalgenerator einen Frequenzverschieber auf, welcher also ein im Signalgenerator erzeugtes oder bereitgestelltes Signal (z.B. Sinussignal, schmalbandiges Rauschen, Signalanteil aus Eingangssignal) um die Beatfrequenz verschiebt und damit das frequenzverschobene Beatsignal erzeugt.

Bei dem momentanen Signalpegel des Eingangssignals handelt es sich allgemein um einen Kennwert für den Signalpegel, also für die Amplitude des akustischen oder auch des gestreamten Eingangssignals, welches vom Eingangswandler erfasst wird. Die Bestimmung des momentanen Signalpegels erfolgt dabei regelmäßig auf Basis einer Auswertung des gewandelten, elektrischen Eingangssignals. Speziell handelt es sich bei der Bestimmung des momentanen Signalpegels um eine Schätzung. Mit der Bestimmung des momentanen Signalpegels wird daher insgesamt ein Signalpegel und damit eine Amplitude der momentanen Umgebungsgeräusche bestimmt.

In einer bevorzugten Ausgestaltung ist die Einstellvorrichtung dazu eingerichtet, dass der Signalpegel des Beatsignalanteils im akustischen Ausgangssignal um 1 dB bis 5 dB höher ist als der Signalpegel des normalen Audiosignalanteils im akustischen Ausgangssignal. Untersuchungen haben gezeigt, dass eine derartige Wahl besonders geeignet ist, um den gewünschten Effekt der monauralen oder binauralen Beats zu erzeugen, ohne Gefahr zu laufen, dass die (zusätzlichen) Beatsignale als störend wahrgenommen werden.

Zur Bestimmung des momentanen Signalpegels können unterschiedliche Verfahren eingesetzt werden. Beispielsweise wird ein breitbandiger Signalanteil des Eingangssignals, beispielsweise die komplette Bandbreite des hörbaren Frequenzspektrums oder nur ein Teilband ausgewertet. Hierbei werden beispielsweise die Signalpegel in verschiedenen Frequenzbändern gemittelt und der gemittelte Signalpegel wird als der Signalpegel des Eingangssignals betrachtet. Alternativ wird das Frequenzband mit dem höchstem Signalpegel herangezogen und dieser maximale Signalpegel wird als der Signalpegel des Eingangssignals betrachtet

Auch besteht die Möglichkeit, eine Referenzfrequenz festzulegen, bei der der Signalpegel als Referenzpegel und damit als momentaner Signalpegel bestimmt wird. Bei der Referenzfrequenz handelt es sich beispielsweise um eine mittlere Frequenz eines ausgewählten Frequenzbandes oder um die Frequenz mit dem höchsten Signalpegel. Das Frequenzband und / oder die Referenzfrequenz werden beispielsweise fest vorgegeben oder alternativ dynamisch jeweils anhand des momentanen maximalen Signalpegels bestimmt.

Gemäß einer bevorzugten Ausgestaltung stimmt die Referenzfrequenz mit einer Beatsignal-Frequenz der Beatsignale überein, zumindest innerhalb eines Toleranzbereiches von beispielsweise +/ - 30 Hz.

Unter Beatsignal-Frequenz wird zunächst eine mittlere Frequenz der beiden zueinander frequenzverschobenen Beatsignale verstanden. Bestehen die Beatsignale aus einem einzelnen Ton (Sinussignal), so wir die Frequenz des Sinussignals herangezogen. Weist das jeweilige Beatsignal ein Frequenzband auf, z.B. bei einem schmalbandigen Rauschen, so wird für die Beatsignal-Frequenz die mittlere Frequenz des Frequenzbandes herangezogen.

Gemäß einer bevorzugten Alternative wird zwischen der Referenzfrequenz und der Beatsignal-Frequenz bewusst ein Offset eingestellt, um Maskierungseffekte zu vermeiden. Der Offset liegt beispielsweise im Bereich von 50Hz bis 100 Hz. Bevorzugt ist die Referenzfrequenz fest vorgegeben. Alternativ wird die Referenzfrequenz jeweils dynamisch speziell auf Basis der Frequenz eines maximalen Signalpegels bestimmt und die Beatsignal-Frequenz wird ebenfalls dynamisch angepasst.

In bevorzugter Ausgestaltung weist zumindest eines der Hörgeräte einen Synchronisierer auf, welcher dazu eingerichtet ist, anhand der von den beiden Analyseeinheiten ermittelten momentanen Signalpegel ein synchronisiertes Einstellsignal zu erzeugen, mit dem der jeweilige Signalpegel des jeweiligen Beatsignals über das jeweilige Verstärkerelement in dem jeweiligen Hörgerät eingestellt wird. Es erfolgt mittels des Synchronisierers daher eine abgestimmte, synchronisierte Einstellung der Verstärkungsfaktoren für die beiden Beatsignale. Der Synchronisierer vergleicht hierzu beispielsweise die beiden momentanen Signalpegel und entscheidet anhand des Vergleichs, welcher Verstärkungsfaktor für das jeweilige Beatsignal eingestellt wird.

Gemäß einer Ausführungsvariante ist dabei vorgesehen, dass die beiden Signale derart verstärkt werden, dass für die Kanäle unterschiedliche Verhältnisse zwischen dem Signalpegel des jeweiligen Eingangssignals und des jeweiligen Beatsignals eingestellt werden. Speziell sind daher die Unterschiede (Pegelabstand) zwischen dem jeweiligen momentanen Signalpegel und dem zugehörigem Signalpegel des zugehörigen Beatsignals bei den beiden Beatsignalen verschieden. Dies ist insbesondere bei stark unsymmetrischen Audioquellen von Vorteil, wenn also in einer bestimmten Hörsituation eine dominante seitliche akustische Quelle vorliegt. Durch die Synchronisation und geeignete Anpassung der Verstärkungsfaktoren in den beiden Hörgeräten wird beispielsweise erreicht, dass die Signalpegel der Beatsignale nicht zu sehr auseinanderlaufen, also nicht zu sehr verschieden sind, da ein großer Unterschied zwischen den Signalpegeln der Beatsignale die Sinneswahrnehmung der Beats beeinträchtigt. Durch den Synchronisierer werden daher die beiden Beatsignale im Hinblick auf ihre Signalpegel aufeinander abgestimmt.

In bevorzugter Ausgestaltung wird der jeweilige momentane Signalpegel wiederkehrend innerhalb eines vorgegebenen Zeitintervalls bestimmt und der Signalpegel des Beatsignals wird wiederkehrend in Abhängigkeit des momentanen Signalpegels eingestellt. Das Zeitintervall beträgt dabei vorzugsweise maximal 300 ms und insbesondere maximal 100 ms. Dadurch ist eine schnelle und möglichst Verzögerungsfreie Nachführung des Signalpegels der Beatsignale an den momentanen Signalpegel gewährleistet, was zu einer angenehmen Sinneswahrnehmung der Beats führt.

Innerhalb der Signalverarbeitungseinheit, über die das elektrische Eingangssignal verarbeitet wird, ist regelmäßig ein Signalverstärker integriert, welcher das typischerweise bereits teilweise aufbereitete elektrische Eingangssignal verstärkt, und zwar üblicherweise nutzerspezifisch in Abhängigkeit des nutzerspezifischen Hörschadens. Der Signalverstärker weist üblicherweise auch eine Kompressionseinheit auf, die das Signal komprimiert. Gemäß einer bevorzugten Ausgestaltung ist der Summierer vor diesem Signalverstärker angeordnet, sodass also das Beatsignal zusammen mit dem teilweise aufbereiteten elektrischen Eingangssignal gemeinsam verstärkt wird. Dies hat den Vorteil, dass bestimmte vorteilhafte Funktionen des Signalverstärkers, wie beispielsweise eine Pegelbegrenzung für das vom Ausgangswandler abgegebene akustische Ausgangssignal, für das summierte Signal verwendet werden.

Gemäß einer alternativen Ausführungsvariante ist der Summierer nach einem solchen Signalverstärker angeordnet.

In bevorzugter Ausgestaltung weist das Hörsystem ein nutzerspezifisches Einstellelement auf, über welches eine manuelle Einstellung des Beatsignals, insbesondere des Signalpegels des Beatsignals möglich ist. Das Hörsystem weist hierzu beispielsweise eine Bedien-Schnittstelle auf, insbesondere in Form eines tragbaren Gerätes wie beispielsweise ein Smartphone, auf dem eine Anwendung installiert ist, über die Einstellungen für das jeweilige Hörgerät vorgenommen werden können. Dieses tragbare Gerät ist dabei Teil des Hörsystems. Alternativ oder ergänzend können auch unmittelbar an zumindest einem Hörgerät Bedienelemente angeordnet sein, über die die Einstellung erfolgen kann.

Über diese manuellen Einstellmöglichkeiten bietet sich daher für den Nutzer die Möglichkeit, Parameter, welche die Beats beeinflussen selber zumindest nach zu justieren, um ein möglichst angenehmes Hörerempfinden zu erhalten. Die Parameter sind dabei insbesondere die Beatfrequenz und / oder der Verstärkungsfaktor für das Verstärkerelement bzw. der Signalpegel des jeweiligen Beatsignals, insbesondere der Pegelabstand im Vergleich zum normalen Audiosignalanteil im akustischen Ausgangssignal.

In bevorzugter Weiterbildung weist das Hörsystem einen Statuserkenner auf, ist also zur automatischen Erkennung eines momentanen Status des Nutzers ausgebildet. Weiterhin ist das Hörsystem, insbesondere die Einstellvorrichtung dazu eingerichtet, in Abhängigkeit des identifizierten momentanen Status das zumindest eine Beatsignal einstellt. Unter Status des Nutzers wird insbesondere ein momentaner körperlicher Zustand des Nutzers verstanden, welcher beispielsweise durch eine Bestimmung des momentanen Pulses des Nutzers und/oder durch Beschleunigungssensoren ermittelt wird. Die Bestimmung des momentanen Status kann dabei entweder mit Hilfsgeräten oder direkt auch mithilfe des Hörgeräts vorgenommen werden. Beispielsweise können im Hörgerät Beschleunigungssensoren integriert sein oder das Hörgerät ist dazu ausgelegt, aus akustischen Signalen, z.B. Körperschall-Signale, den momentanen Puls des Hörgeräteträgers zu bestimmen.

Insbesondere wird ein Schlafmodus identifiziert, insbesondere im Vergleich zu einem Wach-Modus, wobei dann für die unterschiedlichen Modi die Parameter unterschiedlich eingestellt werden. Unter Parameter für das Beatsignal werden wiederum insbesondere die Beatfrequenz (also die Größe der Frequenzverschiebung zwischen den Beatsignalen) und / oder der Signalpegel verstanden. So wird beispielsweise bei einem identifizierten Schlafmodus der Signalpegel im Vergleich zu einem Wach-Modus reduziert. Ergänzend oder alternativ werden für den Wach-modus und dem Schlafmodus unterschiedliche Beatfrequenzen eingestellt. Es ist nämlich bekannt, dass unterschiedliche Beatfrequenzen unterschiedliche Auswirkungen und Trainingseffekte für das Gehirn aufweisen.

In bevorzugter Ausgestaltung wird - beispielsweise im Schlafmodus - die Beatfrequenz auf einen Bereich zwischen 35 Hz bis 45 Hz und insbesondere auf 40 Hz eingestellt. Untersuchungen haben gezeigt, dass ein derartige Beatfrequenzen speziell im Schlafmodus besondere Vorteile aufweisen.

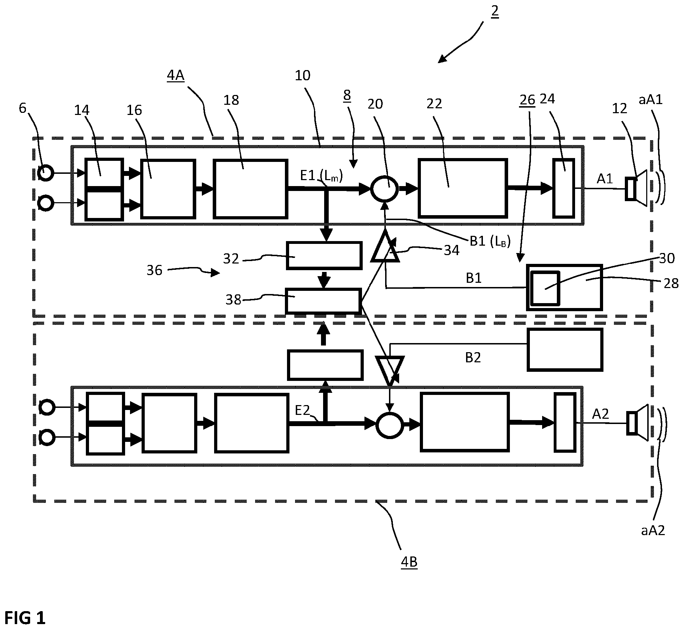

Ein Ausführungsbeispiel der Erfindung wird nachfolgend anhand der Figur näher erläutert. Diese zeigt in einer vereinfachten Blockbild-Darstellungen eine Ausführungsvariante eines binauralen Hörsystems.

Das dargestellte Hörsystem 2 ist als ein Hörhilfesystem ausgebildet, welches zur Kompensation eines benutzerspezifischen Hörschadens eingerichtet ist. Es handelt es sich hierbei um ein binaurales Hörsystem 2 mit zwei Hörgeräten 4A, 4B welche jeweils über eine hier nicht näher dargestellte drahtlose Kommunikationseinheit, insbesondere eine Bluetooth-Kommunikations-Schnittstelle in an sich bekannter Weise miteinander kommunizieren. Die beiden Hörgeräte 4A, 4B sind zumindest weitgehend identisch aufgebaut. Auf Unterschiede wird explizit eingegangen.

Ein jeweiliges Hörgerät 4A, 4B weist jeweils einen oder mehrere Eingangswandler 6 auf, insbesondere Mikrofone, über die ein Eingangssignal, insbesondere ein akustisches Umgebungssignal, in ein elektrisches Eingangssignal E1, E2 umgewandelt wird. Dieses wird in einem Haupt-Signalpfad 8 und in einer Signalverarbeitungseinheit 10 zunächst in an sich bekannter Weise speziell auf Basis eines nutzerspezifischen Audiogramms und damit entsprechend einem nutzerspezifischen Hörschaden aufbereitet. Das aufbereitete elektrische Signal wird als elektrisches Ausgangssignal A1, A2 einem Ausgangswandler 12 zugeführt, welcher das elektrische Ausgangssignal in ein akustisches Ausgangssignal aA1, aA2 wandelt. Bei dem Ausgangswandler 12 handelt es sich insbesondere um einen Lautsprecher.

Die Signalverarbeitungseinheit 10 weist beispielsweise eingangsseitig eine Filterbank 14 auf, über die das elektrische Eingangssignal E1, E2 in unterschiedliche Frequenzbänder für die nachfolgende Signalbearbeitung zerlegt wird. Nachfolgend sind üblicherweise mehrere Signalverarbeitungseinheiten 16,18 wie beispielsweise ein Beamformer zur Erzeugung einer Direktionalität oder auch ein Rauschunterdrücker zur Reduzierung von störenden Umgebungsgeräuschen vorgesehen. Ein durch die Signalverarbeitungseinheiten 16,18 teilaufbereitetes elektrisches Eingangssignal E1, E2 wird anschließend einem Summierer 20 zugeführt. Nachfolgend zu dem Summierer 20 ist ein Signalverstärker 22 typischerweise mit integrierter Kompressionseinheit angeordnet. Dem Signalverstärker 22 ist schließlich beispielsweise eine synthetisierende Filterbank 24 nachgeordnet, die die einzelnen Frequenzbänder wieder zusammenführt und an dessen Ende das elektrische Ausgangssignal A1, A2 ausgegeben wird.

Neben dem Haupt-Signalpfad 8 weist ein jeweiliges Hörgerät 4A, 4B einen Neben-Signalpfad 26 auf. Dieser weist einen Signalgenerator 28 zur Erzeugung eines Beatsignals B1, B2 auf. Über den Signalgenerator 28 wird insbesondere zusätzlich zu dem elektrischen Eingangssignal E1, E2 ein weiteres Signal bereitgestellt. Bei diesem handelt es sich insbesondere um ein vom Eingangssignal unabhängiges Signal, speziell ein Sinussignal oder um ein insbesondere schmalbandiges Rauschsignal. Daneben besteht auch die Möglichkeit, als Basis für das Beatsignal B1, B2 ein Signalanteil des vom Eingangswandler 6 aufgenommen elektrischen Eingangssignals E1, E2, also Umgebungsgeräusche, heranzuziehen. Das Beatsignal B1, B2 setzt sich beispielsweise aus mehreren Signalanteilen zusammen, die auf Basis der oder durch die zuvor erwähnten Signale (Sinussignal, Rauschsignal, Umgebungsgeräusch) gebildet sind.

Zumindest in einem der beiden Signalgeneratoren 28, im Ausführungsbeispiel im ersten Hörgerät 4A, ist ein Frequenzverschieber 30 integriert, mit dessen Hilfe das zuvor generierte Signal (z.B. Sinussignal, Rauschsignal) um eine Beatfrequenz verschoben wird. Bei den beiden Beatsignalen B1, B2 handelt es sich daher um zwei identische Signale mit den gleichen Signalanteilen, die zueinander lediglich um die Beatfrequenz verschoben sind.

Weiterhin weist ein jeweiliges Hörgerät 4A, 4B jeweils eine Analyseeinheit 32 auf, die dazu eingerichtet ist, einen momentanen Signalpegel Lₘ des (akustischen) Eingangssignals zu ermitteln, insbesondere abzuschätzen. Hierzu wertet die Analyseeinheit 32 das vor dem Summierer 20 anliegende und ggf. teilweise aufbereitete elektrische Eingangssignal E1, E2 in geeigneter Weise aus.

In Abhängigkeit des dabei ermittelten momentanen, aktuell anliegenden Signalpegels Lₘ wird ein momentaner Verstärkungsfaktor bestimmt, mit dem das jeweilige Beatsignal B1, B2 verstärkt wird. Zur Verstärkung ist jeweils ein Verstärkerelement 34 angeordnet. Die Analyseeinheit 32 und das Verstärkerelement 34 sind Bauteile einer Einstellvorrichtung 36, über die ein jeweiliger Signalpegel L_{B} für das Beatsignal B1, B2 eingestellt wird.

Gemäß einer bevorzugten Ausgestaltung, wie sie in der Figur dargestellt ist, ist zumindest in einem der Hörgeräte 4A, 4B ein Synchronisierer 38 angeordnet, welcher mit den beiden Analyseeinheiten 32 verbunden ist und von diesen Information über den jeweiligen Signalpegel Lₘ erhält. Die Übermittlung dieser Information von dem einen Hörgerät 4B zu dem im anderen Hörgerät 4A angeordneten Synchronisierer 38 erfolgt insbesondere über die zuvor beschriebene Kommunikation-Schnittstelle des binauralen Hörsystems 2.

Der Synchronisierer 38 wertet die beiden momentanen Signalpegel Lₘ aus, vergleicht sie insbesondere und ermittelt auf Basis dieser Auswertung geeignete, synchronisierte Verstärkungsfaktoren für die beiden Beatsignale B1, B2. Bei stark unterschiedlichen momentanen Signalpegel Lₘ sorgt der Synchronisierer 38 insbesondere für eine Begrenzung der Unterschiede der Signalpegel L_{B} der Beatsignale B1, B2.

In einer alternativen, vereinfachten und hier nicht näher dargestellten Ausführungsvariante bestimmt jede Einstellvorrichtung 36 der beiden Hörgeräte 4A, 4B individuell einen jeweiligen momentanen Verstärkungsfaktor für das jeweilige Beatsignal B1, B2. Bei dieser Variante wird daher auf den Synchronisierer 38 und eine Synchronisation verzichtet.

Das jeweils verstärkte Beatsignal B1, B2 wird anschließend dem Summierer 20 zugeführt. Nachfolgend wird das resultierende, zusammengesetzte Signal, welches als Signalanteile das teilweise aufbereitete Eingangssignal E1, E2 sowie das Beatsignal B1, B2 aufweist, dem Signalverstärker 22 zugeführt, dort verstärkt und schließlich nach der synthetisierenden Filterbank 24 als das ausgehende elektrische Ausgangssignal A1, A2 bereitgestellt, welches anschließend durch den Ausgangswandler 12 in das akustische Ausgangssignal aA1, aA2 umgewandelt wird.

Das jeweilige akustische Ausgangssignal aA1, aA2 setzt sich daher aus einem normalen Audiosignalanteil und einem Beatsignalanteil zusammen. Der normale Audiosignalanteil, ist der Signalanteil, welcher sich ohne den jeweiligen Signalanteil der Beatsignale ergeben würde. Die Beatsignalanteile im akustischen Ausgangssignal aA1, aA2 sind zueinander um die Beatfrequenz frequenzverschoben und weisen ggf. unterschiedliche Amplituden auf, sind ansonst jedoch identisch.

Dem Nutzer werden daher an seinen beiden Ohren zusätzlich zu dem normalen Audiosignal zwei zueinander um die Beatfrequenz versetzte (akustische) Beatsignale B1, B2 bereitgestellt, so dass wie gewünscht die binauralen Beats vom Nutzer wahrgenommen werden.

Der momentane Signalpegel Lₘ wird vorzugsweise fortlaufend und vorzugsweise in Zeitintervallen von kleiner 300ms und beispielsweise alle 100ms erfasst und der Signalpegel L_{B} der Beatsignale B1, B2 wird kontinuierlich anhand der erfassten momentanen Signalpegel Lₘ nachgeführt. Und zwar vorzugsweise derart, dass sich im akustischen Ausgangssignal aA1, aA2 ein höherer Signalpegel L_{B} der Beatsignal-Anteile im Vergleich zu den Signalpegeln Lₘ des normalen Audiosignalanteils einstellt. Bevorzugt ist der Signalpegel L_{B} der Beatsignalanteile um 1dB bis 5dB und insbesondere um 3dB höher.

Vorliegend werden die elektrischen Eingangssignale über den ganzen Haupt-Signalpfad 8 einheitlich - unabhängig von ihrem Bearbeitungsstatus - mit dem Bezugszeichen E1, E2 versehen.

Vorliegend sind die vom jeweiligen Signalgenerator 28 bereitgestellten weiteren (Beat-) Signale über den gesamten Signalpfad und damit in unterschiedlichen Bearbeitungsstufen einheitlich mit dem jeweiligen Bezugszeichen B1, B2 bezeichnet.

Bei den zuvor angeführten Einheiten wie Filterbank 14, 24, Signalverarbeitungseinheit 16, 18, Summierer 20, Signalverstärker 22, Signalgenerator 28, Frequenzverschieber 30, Analyseeinheit 32, Verstärkerelement 34 sowie Synchronisierer 38 handelt es sich jeweils um elektronische Bauteile oder Gruppen von elektronischen Schaltungsbauteilen, die im Betrieb die gewünschten beschriebenen Funktionen umsetzen. Diese Einheiten sind beispielsweise in einem oder in mehreren integrierten Schaltkreisen, Mikrochips und/oder Asics integriert.

### Bezugszeichenliste

- 2: Hörsystem
- 4A, B: Hörgerät
- 6: Eingangswandler
- 8: Haupt-Signalpfad
- 10: Signalverarbeitungseinheit
- 12: Ausgangswandler
- 14: Filterbank
- 16: Signalverarbeitungseinheit
- 18: Signalverarbeitungseinheit
- 20: Summierer
- 22: Signalverstärker
- 24: synthetisierende Filterbank
- 26: Neben-Signalpfad
- 28: Signalgenerator
- 30: Frequenzverschieber
- 32: Analyseeinheit
- 34: Verstärkerelement
- 36: Einstellvorrichtung
- 38: Synchronisierer

- E1, E2: elektrisches Eingangssignal
- A1, A2: elektrisches Ausgangssignal
- aA1, aA2: akustisches Ausgangssignal
- B1, B2: Beatsignal
- Lₘ: Signalpegel des Eingangssignals
- L_{B}: Signalpegel des Beatsignals

## Patentansprüche

1. Hörsystem (2), insbesondere binaurales Hörsystem (2), welches zur Erzeugung von monauralen oder binauralen Beats ausgebildet ist, mit zumindest einem Hörgerät (4A, 4B), wobei
- das zumindest eine Hörgerät (4A, 4B) einen Haupt-Signalpfad (8) aufweist, mit einem Eingangswandler (6) zur Erzeugung eines elektrischen Eingangssignals (E1, E2) aus einem insbesondere akustischen Eingangssignal, mit einer Signalverarbeitungseinheit (10) zur Verarbeitung des elektrischen Eingangssignals (E1, E2) und zur Erzeugung eines elektrischen Ausgangssignals (A1, A2), sowie mit einem Ausgangswandler (12), welcher basierend auf dem elektrischen Ausgangssignal (A1, A2) ein akustisches Ausgangssignal (aA1, aA2) erzeugt,
- das zumindest eine Hörgerät (4A, 4B) weiterhin einen Neben-Signalpfad (26) aufweist, mit einem Signalgenerator (28) zur Erzeugung eines ersten Beatsignals (B1), wobei das erste Beatsignal (B1) zu einem weiteren Signal (B2) um eine Beatfrequenz frequenzverschoben ist, derart, dass im Betrieb beim Nutzer der Effekt des monauralen oder binauralen Beats erzeugt wird,
- das zumindest eine Hörgerät (4A, 4B) einen Summierer (20) aufweist, welcher zumindest das erste Beatsignal (B1) auf ein Signal des Haupt-Signalpfades (8) aufsummiert, **dadurch gekennzeichnet, dass**
- dem Signalgenerator (28) eine Einstellvorrichtung (36) zugeordnet ist, die ein einstellbares Verstärkerelement (34) aufweist, wobei die Einstellvorrichtung (36) eine Analyseeinheit (32) zur Bestimmung eines momentanen Signalpegels (Lₘ) des Eingangssignals (E1, E2) aufweist, und dass die jeweilige Einstellvorrichtung (36) dazu eingerichtet ist, einen Signalpegel (L_{B}) des ersten Beatsignals (B1) in Abhängigkeit des momentanen Signalpegels (Lₘ) einzustellen.

2. Hörsystem (2) nach dem vorhergehenden Anspruch, welches als binaurales Hörsystem (2) und zur Erzeugung von binauralen Beats ausgebildet ist, mit dem zumindest einen, ersten Hörgerät (4A) und mit einem zweiten Hörgerät (4B), die für eine drahtlose, binaurale Kommunikation miteinander ausgebildet sind, wobei
- jedes der Hörgeräte (4A, 4B) einen Haupt-Signalpfad (8) aufweist, mit einem Eingangswandler (6) zur Erzeugung eines elektrischen Eingangssignals (E1, E2), mit einer Signalverarbeitungseinheit (10) zur Verarbeitung des Eingangssignals (E1, E2) und zur Erzeugung eines elektrischen Ausgangssignals (A1, A2), sowie mit einem Ausgangswandler (12), welcher basierend auf dem elektrischen Ausgangssignal (A1, A2) ein akustisches Ausgangssignal (aA1, aA2) erzeugt,
- jedes der Hörgeräte (4A, 4B) weiterhin einen Neben-Signalpfad (26) aufweist, mit jeweils einem Signalgenerator (28) zur Erzeugung des ersten Beatsignals (B1) im ersten Hörgerät (4A) und eines zweiten Beatsignals (B2) im zweiten Hörgerät (4B), wobei die beiden Beatsignale (B1, B2) zueinander um eine Beatfrequenz frequenzverschoben sind, derart, dass im Betrieb beim Nutzer der Effekt des binauralen Beats erzeugt wird,
- jedes der Hörgeräte (4A, 4B) einen Summierer (20) aufweist, welcher die Beatsignale (B1, B2) auf ein Signal des Haupt-Signalpfades (8) aufsummiert, so dass das akustische Ausgangssignal (aA1, aA2) jeweils gebildet ist durch einen normalen Audiosignalanteil und einem Beatsignalanteil, **dadurch gekennzeichnet, dass**
- dem jeweiligen Signalgenerator (28) jeweils eine Einstellvorrichtung (36) zugeordnet ist, die ein einstellbares Verstärkerelement aufweist, wobei die Einstellvorrichtung (36) zumindest eines der Hörgeräte (4A, 4B) eine Analyseeinheit (32) zur Bestimmung eines momentanen Signalpegels (Lₘ) des Eingangssignals (E1, E2) aufweist, und dass die jeweilige Einstellvorrichtung (36) dazu eingerichtet ist, einen Signalpegel (L_{B}) der Beatsignale (B1, B2) in Abhängigkeit des momentanen Signalpegels (Lₘ) einzustellen.

3. Hörsystem (2) nach einem der vorhergehenden Ansprüche, **dadurch gekennzeichnet, dass** die Einstellvorrichtung (36) dazu eingerichtet ist, dass im akustischen Ausgangssignal (aA1, aA2) der Signalpegel (L_{B}) des Beatsignalanteils um 1 dB bis 5 dB höher ist als der Signalpegel (Lₘ)) des normalen Audiosignalanteils.

4. Hörsystem (2) nach einem der vorhergehenden Ansprüche, **dadurch gekennzeichnet, dass** die Einstellvorrichtung (36) dazu eingerichtet ist, den momentanen Signalpegel (Lₘ) bei einer Referenzfrequenz zu ermitteln und dass eine Beatsignal-Frequenz einen Offset zu der Referenzfrequenz aufweist.

5. Hörsystem (2) nach einem der vorhergehenden Ansprüche, **dadurch gekennzeichnet, dass** es zwei Hörgeräte (4A, 4B) aufweist und jedes Hörgerät (4A, 4B) eine Analyseeinheit (32) sowie ein Verstärkerelement (34) aufweist, und dass zumindest eines der Hörgeräte (4A, 4B) einen Synchronisierer (38) aufweist, der dazu eingerichtet ist, anhand der von den beiden Analyseeinheiten (32) bestimmten momentanen Signalpegel (Lₘ) ein synchronisierte Einstellung der Singalpegel (L_{B}) für das jeweilige Beatsignal (B1, B2) an die beiden Verstärkerelemente (34) abzugeben.

6. Hörsystem (2) nach einem der vorhergehenden Ansprüche, **dadurch gekennzeichnet, dass** die jeweilige Einstellvorrichtung (36) dazu eingerichtet ist, den Signalpegel (L_{B})) des Beatsignals (B1, B2) wiederkehrend innerhalb eines Zeitintervalls von maximal 300ms und vorzugsweise von maximal 100ms in Abhängigkeit des momentanen Signalpegels (Lₘ) des akustischen Ausgangssignals (aA1, aA2) einzustellen.

7. Hörsystem (2) nach einem der vorhergehenden Ansprüche, **dadurch gekennzeichnet, dass** die Signalverarbeitungseinheit (10) einen Signalverstärker (22) aufweist und der Summierer (20) vor dem Signalverstärker (22) angeordnet ist.

8. Hörsystem (2) nach einem der vorhergehenden Ansprüche, **dadurch gekennzeichnet, dass** es für eine manuelle Einstellung des zumindest einen Beatsignals (B1, B1) durch den Nutzer ausgebildet ist

9. Hörsystem (2) nach einem der vorhergehenden Ansprüche, **dadurch gekennzeichnet, dass** es zur automatischen Identifizierung eines momentanen Status des Nutzers, insbesondere einen Schlafzustand des Nutzers ausgebildet ist und dass es zur Einstellung des zumindest einen Beatsignals (B1, B2) in Abhängigkeit des identifizierten momentanen Status ausgebildet ist.

10. Hörsystem (2) nach dem vorhergehenden Anspruch, **dadurch gekennzeichnet, dass** die Beatfrequenz in einem identifizierten Schlafmodus auf einen Bereich von 35Hz bis 45 Hz und insbesondere auf 40 Hz eingestellt wird.

11. Verfahren zur Erzeugung von monauralen oder binauralen Beats bei einem Hörsystem (2) mit zumindest einem Hörgerät (4A, 4B), wobei
- das zumindest eine Hörgerät (4A, 4B) einen Haupt-Signalpfad (8) aufweist, mit einem Eingangswandler (6) welcher ein elektrisches Eingangssignal (E1, E2) aus einem insbesondere akustischen Eingangssignal erzeugt, mit einer Signalverarbeitungseinheit (10), die das elektrische Eingangssignal (E1, E2) verarbeitet und ein elektrisches Ausgangssignal (A1, A2) erzeugt, sowie mit einem Ausgangswandler (12), welcher basierend auf dem elektrischen Ausgangssignal (A1, A2) ein akustisches Ausgangssignal (aA1, aA2) erzeugt,
- das zumindest eine Hörgerät (4A, 4B) weiterhin einen Neben-Signalpfad (26) aufweist, mit jeweils einem Signalgenerator (28), welcher ein erstes Beatsignal erzeugt, wobei das erste Beatsignal zu einem weiteren Signal um eine Beatfrequenz frequenzverschoben ist, derart, dass im Betrieb beim Nutzer der Effekt des monauralen oder binauralen Beats erzeugt wird,
- das zumindest eine Hörgerät (4A, 4B) einen Summierer (20) aufweist, welcher zumindest das erste Beatsignal auf ein Signal des ersten Signalpfades aufsummiert, **dadurch gekennzeichnet, dass**
- ein Signalpegel (L_{B}) des Beatsignals in Abhängigkeit eines momentanen Signalpegels (Lₘ) des Eingangssignals (E1, E2) eingestellt wird.
